# EUROPEAN PATENT SPECIFICATION

(11) **EP 2 654 020 B1**
(45) Date of publication and mention of the grant of the patent: **01.08.2018**
(21) Application number: 13161540.3
(22) Date of filing: 28.03.2013
(51) Int. Cl.: G06T 7/73, G06K 9/62, G06K 9/00

(54) **Method and system for determining the direction of the head of a person**
Methode und System zur Bestimmung der Kopfhaltung einer Person
Méthode et système pour la détermination d'orientation de la tête d'une personne

(30) Priority: 16.04.2012 SE 1250377
(43) Date of publication of application: 23.10.2013
(73) Proprietor: Scania CV AB, 151 87 Södertälje (SE)
(72) Inventor: Olofsson, Jonatan, 415 01 Göteborg (SE); Ah-King, Joseph, 151 60 Södertälje (SE)
(74) Representative: Scania CV AB

(56) References cited:
- WO-A2-2004/111687
- SOUMITRY J RAY ET AL: "Coarse head pose estimation of construction equipment operators to formulate dynamic blind spots", ADVANCED ENGINEERING INFORMATICS, ELSEVIER, AMSTERDAM, NL, vol. 26, no. 1, 25 September 2011 (2011-09-25), pages 117-130, XP028339513, ISSN: 1474-0346, DOI: 10.1016/J.AEI.2011.09.005 [retrieved on 2011-10-04]

## Description

### TECHNICAL FIELD OF THE INVENTION

The invention concerns a method for determining the direction of the head of a person according to the preamble to claim 1. The invention also concerns a system for determining the direction of the head of a person according to the preamble to claim 5. The invention also concerns a motor vehicle. The invention further concerns a computer program and a computer program product.

### BACKGROUND

Inattention of vehicle driver is often a contributing factor in traffic accidents. Inattention of the driver can be traced to distraction in the form of cognitive distraction or visual distraction. Inattention of the driver can also be attributable to tiredness. Inattention on the part of the driver can be attributable to a combination of distraction and tiredness. A multitude of studies have been performed regarding the behavior of the driver in connection with inattention. One behavior that can be traced to inattention of the aforementioned type consists in that the driver turns his head to, for example, the left or right, e.g. due to driver distractions such as changing the heat setting, talking on the telephone, lifting and consuming food/snacks, movable objects etc.

A number of systems for monitoring driver inattention are known. One means of detecting the head direction of a vehicle driver is to use a CCD camera that detects luminous intensity, wherein the camera detects the position and angle of the head of the driver, whereupon if the head of the driver is pointed in a given direction and an obstacle is detected, a warning system is activated.

Other systems for monitoring driver inattention are known in which the detection of an eye direction for assessing inattention is achieved through the utilization of a plurality of cameras and IR technology.

Other approaches are known from e.g. Soumitry, J. R. and Teizer, J., "Coarse head pose estimation and construction equipment operators to formulate dynamic blind spots", Advanced Engineering Informatics, Vol. 26, No. 1, pp. 117-130, 2012, or WO 2004/111687 A2.

One problem with utilizing luminous intensity to determine the direction of the head is that the precision of the calculation of the direction of the head is limited.

### OBJECTS OF THE INVENTION

One object of the present invention is to provide a method for determining the direction of the head of a person, which method is of high precision and does not require specific information about the head shape.

One object of the present invention is to provide a system for determining the direction of the head of a person, which method is of high precision and does not require specific information about the head shape.

### SUMMARY OF THE INVENTION

These and the other objects, which are presented in the description below, are achieved by means of a method, a system, a motor vehicle, a computer program and a computer program product of the kind specified above, and which further exhibits the features specified in the characterizing portion of attached independent claims 1, 9, 17, 18 and 19. Preferred embodiments of the method and the system are described in attached non-independent claims 2-8 and 10-16.

According to the invention, these objects are achieved by a method for determining the direction of the head of a person, which method comprises the step of creating an image of said head comprising a desired view of the head, which method comprises the steps of: creating a deep data image of said head; extracting deep data from defined points on the deep data image of the head; and using said extracted deep data for assessing the direction of said head by utilizing a classification algorithm. Precise determinations of the direction of the head of a person are thereby enabled, wherein the determination can be made based on general cases and, consequently, directions can be determined for heads of highly various shapes. This is made possible in part because said deep data from said defined points contain more relevant information for determining the direction of the head than, for example, a slide, whereupon the precision and consequently the capacity for correct determination of the direction of the head of a person are significantly improved and can be applied to general cases without any specific information about the shape of the face, placement of the eyes, nose and/or mouth being required.

According to one embodiment of the method, said classification algorithm is improved continuously during a learning phase with regard to said determination, in that determinations are made, under feedback, for a number of different head shapes in order to adapt the determinative capacity of the algorithm to any head shape. The determination of the direction of the head by means of said deep data from defined points is thereby improved.

According to one embodiment of the method, said classification algorithm is based on characteristics of artificial neural networks. Using an artificial neural network enables effective improvement of said classification algorithm under said learning phase.

According to one embodiment of the method, said classification of the direction of the head comprises essentially straight ahead, turned to the right, turned to the left, turned upward and turned downward. Clear definitions of the head direction are thereby provided to facilitate said determination of the direction of the head.

According to one embodiment, the method further comprises the step of determining the position of said head in the created deep data image. The creation of a deep data image of the head is thereby facilitated.

According to one embodiment of the method, the step of determining the position of said head in the created deep data image comprises the step of creating a two-dimensional head contour as the basis for said determination. The effective creation of a deep data image for effective extraction of deep data from defined points on the deep data image of the head is thereby enabled.

According to one embodiment of the method, said head belongs to a vehicle driver present in a vehicle driver compartment. Thus determining the direction of the head of a vehicle driver enables effective determination of any inattention on the part of the driver that could affect the ability of the driver to operate the vehicle safety, whereupon measures can be taken to alert the driver to the prevailing risk. The inattention can, for example, be attributable to tiredness on the part of the driver.

According to one embodiment of the method, information about said direction of the head and changes therein is used to determine the presence of inattention on the part of said person. The information about inattention can thereby be used to alert the person so as to thus prevent a mistake from being made by the person, prevent an accident and/or improve the performance of the person.

According to the invention, these objects are achieved by a system for determining the direction of the head of a person, which system comprises means for creating an image of said head comprising a desired view of the head, comprising means for creating a deep data image of said head; means for extracting deep data from defined points on the deep data image of the head; means for using said extracted deep data to determine the direction of said head via the utilization of a classification algorithm. Precise determinations of the direction of the head of a person are thereby enabled, whereupon the determination can be made based on general cases and, consequently, the directions of heads with extremely various head shapes can be determined. This is made possible in part in that said deep data from said defined points contain more relevant information for determining the direction of the head than, for example, a slide, whereupon the precision and consequently the capacity to correctly determine the direction of the head of a person are significantly improved and can be applied to general cases without any specific information about the shape of the face, placement of the eyes, nose and/or mouth being required.

According to one embodiment of the system, said classification algorithm is arranged so as, during a learning phase, to continuously improve in terms of said determination in that determinations are made, under feedback, for a number of different head shapes in order to adapt the determinative capacity of the algorithm to any head shape. The determination of the direction of the head by means of said deep data from defined points is thereby improved.

According to one embodiment of the system, said classification algorithm is based on characteristics of artificial neural networks. Using an artificial neural network enables effective improvement of said classification algorithm during said learning phase.

According to one embodiment of the system, said classification of the direction of the head comprises essentially straight ahead, turned to the right, turned to the left, turned upward and turned downward. Clear definitions of head direction are thereby provided to facilitate said determination of the direction of the head.

According to one embodiment, the system further comprises means for determining the position of said head in the created deep data image. The creation of a deep data image of the head is thereby facilitated.

According to one embodiment of the system, said means for determining said position of the head in the created deep data image comprise means for creating a two-dimensional head contour as a basis for said determination. Effective creation of a deep data image for effective extraction of deep data from defined points in the deep data image of the head is thereby enabled.

According to one embodiment of the system, said head belongs to a vehicle driver present in a vehicle driver compartment. Thus determining the direction of the head of a vehicle driver enables effective determination of any inattention on the part of the driver that could affect the ability of the driver to operate the vehicle safely, whereupon measures can be taken to alert the driver to the prevailing risk. The inattention can be attributable to, for example, tiredness on the part of the driver.

According to one embodiment of the system, information about said direction of the head and changes therein is intended to be used to determine the presence of inattention on the part of said person. The information about inattention can thus be used to alert the person in order to thereby prevent a mistake from being made by the person, prevent an accident and/or improve the performance of the person.

### FIGURE DESCRIPTION

The present invention will be better understood with reference to the following detailed description, read together with the attached drawings, wherein the same reference designations consistently refer to the same parts in the many views, and in which:
Fig. 1 schematically illustrates a motor vehicle according to one embodiment of the present invention;
Fig. 2 schematically illustrates a system for determining the direction of the head of a person according to one embodiment of the present invention;
Fig. 3 schematically illustrates a template of a generic head;
Figs. 4a-c schematically illustrate deep data images of persons who are looking in different directions, which images comprise defined points from which deep data are extracted;
Fig. 5 schematically illustrates a block diagram of a method for determining the direction of the head of a person according to one embodiment of the present invention; and
Fig. 6 schematically illustrates a computer according to one embodiment of the present invention.

### DESCRIPTION OF EMBODIMENTS

The term "link" refers herein to a communications link, which can be a physical line, such as an opto-electronic communication line, or a non-physical line, such as a wireless connection, for example a radio or microwave link.

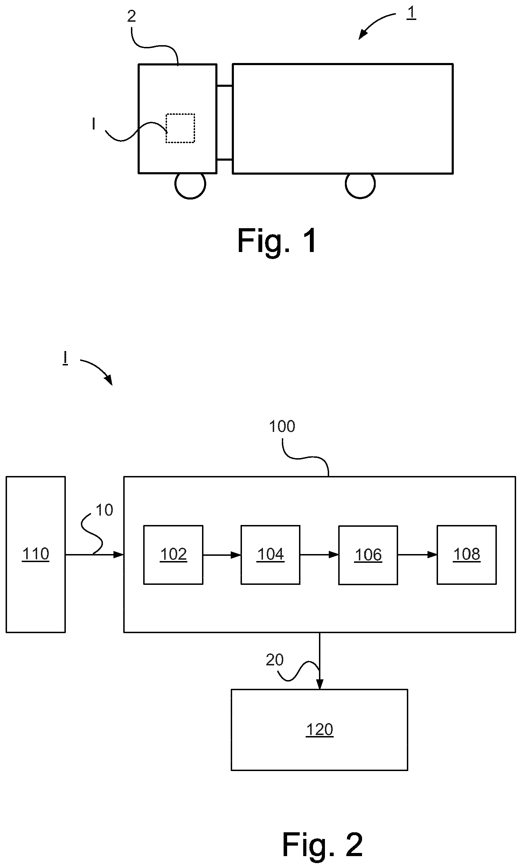

Fig. 1 schematically illustrates a motor vehicle 1 according to one embodiment of the present invention. The exemplary vehicle 1 consists of a heavy vehicle in the form of a goods vehicle with a cab 2. The vehicle can alternatively consist of any suitable vehicle, such as a bus or a car. The vehicle contains a system I according to the present invention.

Fig. 2 schematically illustrates a system I for determining the direction of the head of a person according to one embodiment of the present invention.

The system I comprises an electronic control unit 100 or computer for said determination of the direction of the head a person.

The system I further comprises means 110 for creating a deep data image. Said means 110 for creating a deep data image comprise a camera device 110 for deep data imaging. According to one variant, the camera device for deep data imaging consists of a 3D camera device, wherein the camera device according to one variant consists of a Kinect® from the Microsoft Corporation.

According to one variant, said camera device 110 is arranged in a driver compartment such as a cab. The camera device 100 is herein arranged in the driver compartment to reproduce a deep data image of the head of the driver. The camera device 100 is herein pointed at the face of the driver. According to one variant, the camera device 110 is arranged at a distance from and obliquely above the driver.

The system further comprises means 120 for utilizing information about the direction of the head. Said means 120 can comprise warning devices such as alarm devices, vibrator devices, blinking devices/lighting devices and/or activation devices to activate brake devices on the vehicle to alert the driver. Said warning devices can be dependent upon other information and activated when certain criteria are met, such as, when the system is disposed in a vehicle, an obstacle along the route of the vehicle or the like. Information about the direction of the head and changes therein is consequently, according to this variant, intended to be used to determine the presence of inattention on the part of the person and, by means of said means, to utilize the information to alert the person. The information about inattention can thereby be used to alert the person in order to thus prevent a mistake from being made by the person, prevent an accident, for example, during operation of a vehicle and/or improving the performance of the person.

The electronic control unit 100 is signal-connected to said camera device 110 for deep data imaging via a link 10. The electronic control unit 100 is arranged via the link 10 so as to receive a signal from said camera device 110 representing deep data.

The electronic control unit 100 comprises means 102 for preprocessing said deep data image. Said means 102 for preprocessing said deep data image comprise any suitable image-processing means. According to one variant, said means 102 for preprocessing said deep data image comprise low-pass filtering to reduce noise and to reduce detail in the deep data image. Reduction of detail in the deep data image is performed because only the shape and direction of the head are of interest. According to one variant, said means 102 for preprocessing said deep data image comprise means for creating a contour image of the head comprising an edge detector. According to one variant, a so-called Canny edge detector, which is a previously known algorithm, is used, wherein an image is taken as described with a single value for each pixel, e.g. a gray-scale value or deep value, as input data and performs a number of steps in accordance with the algorithm for producing the contour image.

The electronic control unit 100 comprises means 104 for determining said head position in the created deep data image. Said means 104 for determining said head position in the created deep data image include means for creating a two-dimensional head contour as a basis for said determination. Said means 104 for determining said head position in the created deep data image according to one variant include so-called chamfer matching, which is a previously known algorithm wherein the contour image is utilized to match a binary template to the image. According to one variant, a distance map of the aforesaid created contour image is created, wherein said distance map of the contour image is used to find the position in which the distance for the binary template is minimized.

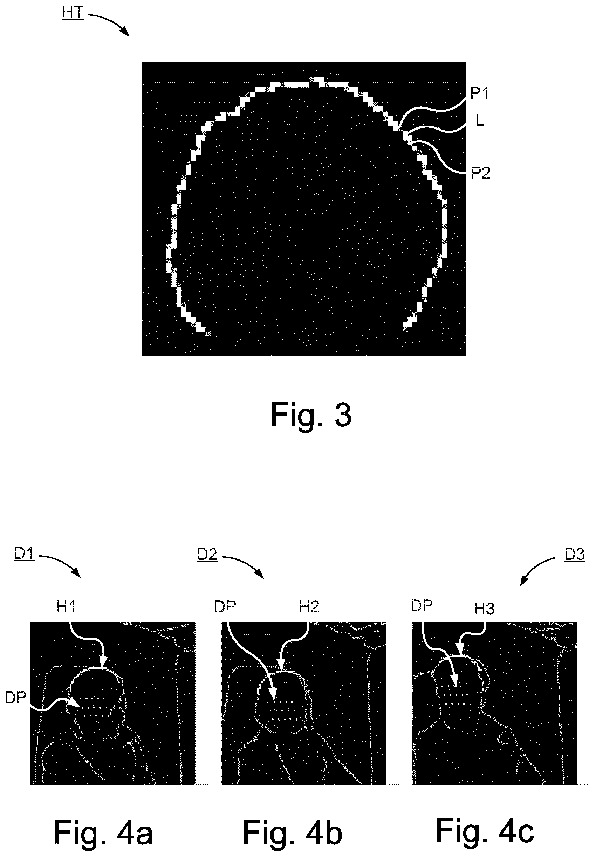

According to one variant, a generic head template is created wherein the matching of the head template is achieved according to one variant by studying how well it matches a selection of a number of different head images of a number of persons, where the persons are looking in different directions in different head images. The performance of the generic head template is intended to be independent of the direction of the head of the person. Fig. 3 shows an example of a generic head template.

The electronic control unit 100 comprises means 106 for extracting deep data from defined points on the deep data image of the head. When the position of the head in the created deep image is known, deep data can be extracted from the deep data image. According to one embodiment, said defined points consist of a set of points distributed according to a predetermined pattern. According to one embodiment shown in Figs. 4a-c, said deep data points DP are divided into three rows, wherein the bottom row is somewhat displaced to the right relative to the overlying rows. According to the embodiment shown in Figs. 4a-c, each row has five points, and the total number of points is consequently fifteen. The number of points can be any suitable number, and the distribution of the points can be any suitable distribution. The coordinates of these rows are calculated based on the known positions for the determined head template.

The electronic control unit 100 comprises means 108 for using said extracted deep data for determining said direction of the head by utilizing a classification algorithm. The fact that said predetermined points of deep data contain information about the head direction is thereby utilized. Said classification algorithm is utilized to find out how said deep data correlate with various directions of the head of a person.

Said classification algorithm is based on characteristics of means for identifying linear relationships between input data in the form of a head shape of a person and output data in the form of various directions of the head of the person, wherein said output data according to one embodiment are classified in that: the person is looking to the left, the person is looking forward, the person is looking to the right.

Said output data can be classified as any suitable direction of the head of the person, including that the person is looking to the left, the person is looking forward, the person is looking to the right, the person is looking upward, the person is looking downward, the person is looking rearward. Said output data can further be classified by a continuous distribution of the head direction, i.e. a continuous distribution of various angles of the head comprising to the left, straight ahead, to the right, rearward and angles therebetween, and/or upward, straight ahead and downward and angles therebetween.

Said classification algorithm is consequently, according to one embodiment, configured so as to identify any distribution of the direction of the head, i.e. to identify the head turned straight forward and any arbitrary angle of the head relative to the head turned straight forward, including the head turned straight forward, the head turned to the: left, right upward, downward, rearward and angles therebetween.

According to one preferred embodiment, said classification algorithm is based on characteristics of artificial neural networks. According to alternative embodiments, said classification algorithm is based on characteristics of a so-called support vector machine, AdaBoost or Bayesian network.

Said classification algorithm is, during a learning phase, arranged so as to be improved continuously in terms of said determination by making, under feedback, determinations for a number of different head shapes in order to adapt the determinative capacity of the algorithm to any head shape. Said input data [are] in the form of head shapes, wherein the shapes during learning consist of head shapes that have essentially different shapes. Furthermore, according to one variant an additional head shape is used for validation.

The artificial neural network is designed to optimize the generalization of the relationship between input data in the form of head shapes and output data in the form of the direction of the head. Two hidden layers, a first hidden layer and a second hidden layer, are a sufficient number of hidden layers, as any continuous or discrete function can be stored in two hidden layers, given that they are sufficiently large. The variables that are taken into account in designing the network are the number of neurons in the first hidden layer, the number of neurons in the second hidden layer, the learning speed η and an amount of motion α.

The electronic control unit 100 is consequently arranged so as, based on said extracted deep data, to determine said direction of the head by utilizing a classification algorithm.

The electronic control unit 100 is signal-connected to said means 120 for utilizing information about the direction of the head via a link 20. The electronic control unit 100 is arranged via the link 20 so as to transmit a signal to said means 120 for utilizing information about the direction of the head representing head direction data from said determined direction of the head for utilizing information about the direction of the head, wherein activation occurs according to the foregoing based on said information, i.e. based on the direction of the head, and on any other information, to alert the person to any inattention.

Fig. 3 schematically illustrates one embodiment of a generic head template HT for optimizing the matching of various head shapes of persons the directions of whose heads are to be determined. The head template HT according to this embodiment comprises a number of distinct points P1, P2, whereupon an algorithm is configured to draw lines L between proximate points P1, P2. Each point is described by an x-coordinate and a γ-coordinate. According to this embodiment, the head template comprises 42 distinct points. The head template HT can have any suitable number of distinct points.

Figs. 4a-c schematically illustrate deep data images D1, D2, D3 of various persons who are looking in various directions, i.e. various directions of the head H1, H2, H3 of various persons with different head shapes, with defined points DP from which deep data are extracted. The persons according to Figs. 4a-c are filtered deep data images D1, D2, D3 of drivers sitting in a driver compartment in a vehicle. The distribution of said points DP from which deep data are extracted therein is three rows of five points, wherein the rows are somewhat mutually displaced so that underlying rows of points are displaced to the right relative to an overlying row of points. This is, as noted, just one example. The points can be arbitrary in number, and distributed in an arbitrary manner.

Fig. 4a shows a driver who is looking to the left, Fig. 4b shows a driver who is looking straight ahead, and Fig. 4c shows a driver who is looking to the right.

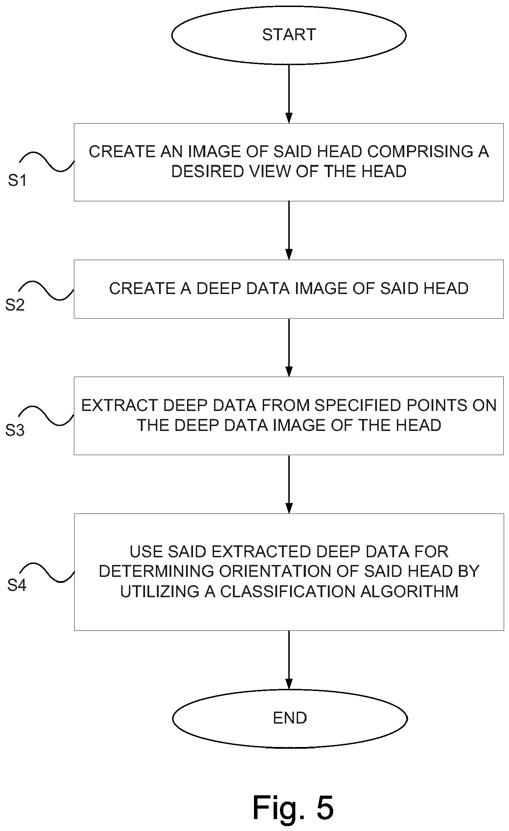

Fig. 5 schematically illustrates a block diagram of a method for determining the direction of the head of a person according to one embodiment of the present invention.

According to one embodiment, the method for determining the direction of the head of a person comprises a first step S1. In this step, an image of said head is created comprising a desired view of the head.

According to one embodiment, the method for determining the direction of the head of a person comprises a second step S2. In this step, a deep data image of said head is created.

According to one embodiment, the method for determining the direction of the head of a person comprises a third step S3. In this step, deep data are extracted from defined points on the deep data image of the head.

According to one embodiment, the method for determining the direction of the head of a person comprises a fourth step S4. In this step, said extracted deep data are used to determine said direction of the head by utilizing a classification algorithm.

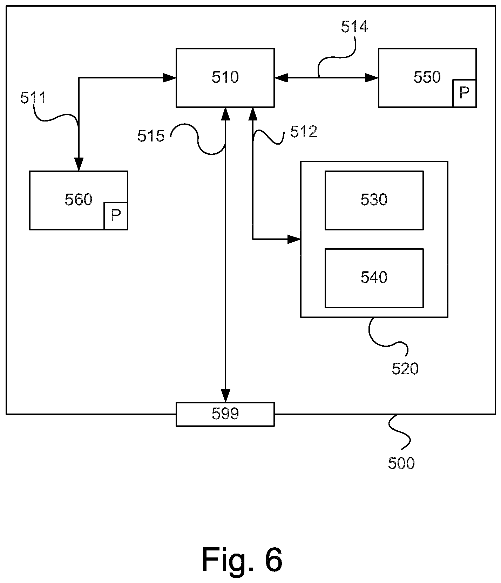

With reference to Figure 6, a diagram is presented of an embodiment of an apparatus 500. The control unit 100, which is described with reference to fig. 2, can, in one embodiment, include the apparatus 500. The apparatus 500 comprises a non-volatile memory 520, a data-processing unit 510 and a read/write memory 550. The non-volatile memory 520 has a first memory section 530 in which a computer program, such as an operating system, is stored to control the function of the apparatus 500. The apparatus 500 further comprises a bus controller, a serial communication port, I/O devices, an A/D converter, the time and date input and transfer unit, an event counter and a termination controller (not shown). The non-volatile memory 520 also has a second memory section 540.

A computer program P is provided that contains routines for determining the direction of the head of a person according to the innovative method. The program P contains routines for creating an image of said head comprising a desired view of the head. The program P contains routines for creating a deep data image of said head. The program P contains routines for extracting deep data from defined points on the deep data image of the head. The program P contains routines for using said extracted deep data for determining said direction of the head by utilizing a classification algorithm. The program P can be stored in an executable form or in a compressed form in a memory 560 and/or in a read/write memory 550.

When it is stated that the data-processing unit 510 performs a given function, it is to be understood that the data-processing unit 510 executes a certain part of the program that is stored in the memory 560, or a certain part of the program that is stored in the read/write memory 550.

The data-processing device 510 can communicate with a data port 599 via a data bus 515. The non-volatile memory 520 is intended to communicate with the data-processing unit 510 via a data bus 512. The separate memory 560 is intended to communicate with the data-processing unit 510 via a data bus 511. The read/write memory 550 is arranged so as to communicate with the data-processing unit 510 via a data bus 514. For example, the links associated with the control unit 100 can be connected to the data port 599.

When data are received at the data port 599, they are stored temporarily in the second memory section 540. Once received input data have been stored temporarily, the data-processing unit 510 is arranged so as to execute code in a manner as described above. The signals received at the data port 599 can be used by the apparatus 500 to create an image of said head comprising a desired view of the head. The signals received at the data port 599 can be used by the apparatus 500 to create a deep data image of said head; extract deep data from defined points on the deep data image of the head. The signals received at the data port 599 can be used by the apparatus 500 to extract deep data from defined points on the deep data image of the head. The signals received at the data port 599 can be used by the apparatus 500 to use said extracted deep data for determining said direction of the head by utilizing a classification algorithm.

Parts of the methods described herein can be performed by the apparatus 500 with the help of the data-processing unit 510, which runs the program stored in the memory 560 or the read/write memory 550. When the apparatus 500 runs the program, the methods described herein are executed.

The foregoing description of the preferred embodiments of the present invention has been furnished for illustrative and descriptive purposes. It is not intended to be exhaustive, or to limit the invention to the variants described. Many modifications and variations will obviously be apparent to one skilled in the art. The embodiments have been chosen and described in order to best explicate the principles of the invention and its practical applications, and to thereby enable one skilled in the art to understand the invention in terms of its various embodiments and with the various modifications that are applicable to its intended use.

## Claims

1. A method for determining information about the direction of the head, wherein said head belongs to a vehicle driver present in a vehicle driver compartment, comprising the steps of
creating (S1) an image of said head comprising a desired view of the head;
creating (S2) a deep data image (D1, D2, D3) of said head;
extracting (S3) deep data from defined points (DP) on the deep data image of the head; **characterized by** the steps of
determining the position of said head in the created deep data image (D1, D2, D3), wherein the step of determining (S4) the position of said head in the created deep data image (D1, D2, D3) comprises the step of creating a two-dimensional head contour as a basis for said determination, wherein said contour image is utilized to match a binary head template,
determining (S4) said direction of the head by utilizing a classification algorithm using said extracted deep data from said defined points (DP) on the deep data image of the head, wherein said classification of the direction of the head comprises essentially straight ahead, turned to the right, turned to the left, turned upward and/or turned downward and wherein said defined points (DP) consists of a set of points distributed according to a predetermined pattern and wherein the coordinates of said defined points (DP) are calculated based on the known positions for the determined head template.

2. A method according to claim 1, wherein said classification algorithm is, during a learning phase, continuously improved in terms of said determination by making, under feedback, determinations for a number of different head shapes in order to adapt the determinative capacity of the algorithm to any head shape.

3. A method according to claim 1 or 2, wherein said classification algorithm is based on characteristics of artificial neural networks.

4. A method according to any of claims 1-3, wherein information about said direction of the head and changes therein is used to determine the presence of inattention on the part of said person.

5. A system (I) for determining information about the direction of the head, wherein said head belongs to a vehicle driver present in a vehicle driver compartment, comprising means (110) for creating an image of said head comprising a desired view of the head, means (100, 110) for creating a deep data image (D1, D2, D3) of said head; means (100, 106) for extracting deep data from defined points (DP) on the deep data image (D1, D2, D3) of the head; means (100, 108) for using said extracted deep data for determining said direction of the head by utilizing a classification algorithm, and wherein said classification of the direction of the head comprises essentially straight ahead, turned to the right, turned to the left, turned upward and/or turned downward **characterized by** that the system further comprising means (100, 104) for determining the position of said head in the created deep data image (D1, D2, D3), wherein said means (100, 104) for determining the position of said head in the created deep data image (D1, D2, D3) comprise means for creating a two-dimensional head contour as a basis for said determination, wherein said contour image is utilized to match a binary template of the image and wherein said defined points (DP) consists of a set of points distributed according to a predetermined pattern and wherein the coordinates of said defined points (DP) are calculated based on the known positions for the determined head template.

6. A system according to claim 5, wherein said classification algorithm is arranged, during a learning phase, so as to be continuously improved in terms of said determination by making, under feedback, determinations for a number of different head shapes in order to adapt the determinative capacity of the algorithm to any head shape.

7. A system according to claim 5 or 6, wherein said classification algorithm is based on characteristics of artificial neural networks.

8. A system according to any of claims 5-7, wherein information about said direction of the head and changes therein is intended to be used to determine the presence of inattention on the part of said person.

9. A motor vehicle (1) containing a system (I) according to any of claims 5-8.

10. A computer program (P) for determining the direction of the head of a person, wherein said computer program (P) contains program code which, when it is run by an electronic control unit (100) or another computer (500) connected to the electronic control unit (100), enables the electronic control unit (100) to perform the steps according to claims 1-4.

11. A computer program product comprising a digital storage medium that stores the computer program (P) according to claim 10.

## Patentansprüche

1. Verfahren zum Ermitteln einer Information über die Richtung des Kopfes, wobei der Kopf zu einem Fahrer eines Fahrzeugs gehört, der in einer Fahrzeugfahrerkabine anwesend ist, wobei das Verfahren Schritte umfasst zum
Erstellen (S1) eines Bilds des Kopfes, das eine gewünschte Ansicht des Kopfes umfasst;
Erstellen (S2) eines tiefgehenden Datenbilds (D1, D2, D3) des Kopfes;
Extrahieren (S3) von tiefgehenden Daten von definierten Punkten (DP) im tiefgehenden Datenbild des Kopfes; **gekennzeichnet durch** die Schritte zum Ermitteln der Position des Kopfes im erstellten tiefgehenden Datenbild (D1, D2, D3), wobei der Schritt zum Ermitteln (S4) der Position des Kopfes im erstellten tiefgehenden Datenbild (D1, D2, D3) den Schritt zum Erstellen einer zweidimensionalen Kopfkontur als eine Basis für die Ermittlung umfasst, wobei das Konturbild eingesetzt wird, um mit einer binären Kopfvorlage übereinzustimmen,
Ermitteln (S4) der Richtung des Kopfes durch Einsetzen eines Klassifizierungsalgorithmus unter Verwendung der extrahierten tiefgehenden Daten von den definierten Punkten (DP) auf dem tiefgehenden Datenbild des Kopfes, wobei die Klassifizierung der Richtung des Kopfes im Wesentlichen geradeaus, nach rechts gedreht, nach links gedreht, nach oben gedreht und/oder nach unten gedreht umfasst, und wobei die definierten Punkte (DP) aus einem Satz von Punkten bestehen, die nach einem vorbestimmten Muster verteilt sind, und wobei die Koordinaten der definierten Punkte (DP) auf Grundlage der bekannten Positionen für die ermittelte Kopfvorlage berechnet werden.

2. Verfahren nach Anspruch 1, wobei der Klassifizierungsalgorithmus während einer Lernphase fortlaufend in Bezug auf die Ermittlung verbessert wird, indem unter Rückmeldung Ermittlungen für eine Anzahl von verschiedenen Kopfformen gemacht werden, um die Ermittlungskapazität des Algorithmus auf eine beliebige Kopfform anzupassen.

3. Verfahren nach Anspruch 1 oder 2, wobei der Klassifizierungsalgorithmus auf Merkmalen von künstlichen neuronalen Netzen basiert.

4. Verfahren nach einem der Ansprüche 1-3, wobei eine Information über die Richtung des Kopfes und Änderungen darin verwendet wird, um das Vorhandensein von Unachtsamkeit auf Seiten der Person zu ermitteln.

5. System (I) zum Ermitteln einer Information über die Richtung des Kopfes, wobei der Kopf zu einem Fahrer eines Fahrzeugs gehört, der in einer Fahrzeugfahrerkabine anwesend ist, umfassend Mittel (110) zum Erstellen eines Bilds des Kopfes, das eine gewünschte Ansicht des Kopfes enthält, Mittel (100, 110) zum Erstellen eines tiefgehenden Datenbilds (D1, D2, D3) des Kopfes; Mittel (100, 106) zum Extrahieren von tiefgehenden Daten von definierten Punkten (DP) im tiefgehenden Datenbild (D1, D2, D3) des Kopfes; Mittel (100, 108) zum Verwenden der extrahierten tiefgehenden Daten zum Ermitteln der Richtung des Kopfes durch Einsetzen eines Klassifizierungsalgorithmus, und wobei die Klassifizierung der Richtung des Kopfes im Wesentlichen geradeaus, nach rechts gedreht, nach links gedreht, nach oben gedreht und/oder nach unten gedreht umfasst, **dadurch gekennzeichnet, dass** das System ferner Mittel (100, 104) zum Ermitteln der Position des Kopfes im erstellten tiefgehenden Datenbild (D1, D2, D3) umfasst, wobei die Mittel (100, 104) zum Ermitteln der Position des Kopfes im erstellten tiefgehenden Datenbild (D1, D2, D3) Mittel zum Erstellen einer zweidimensionalen Kopfkontur als eine Basis für die Ermittlung umfassen, wobei das Konturbild eingesetzt wird, um mit einer binären Kopfvorlage des Bilds übereinzustimmen, und wobei die definierten Punkte (DP) aus einem Satz von Punkten bestehen, die nach einem vorbestimmten Muster verteilt sind, und wobei die Koordinaten der definierten Punkte (DP) auf Grundlage der bekannten Positionen für die ermittelte Kopfvorlage berechnet werden.

6. System nach Anspruch 5, wobei der Klassifizierungsalgorithmus während einer Lernphase ausgelegt ist, fortlaufend in Bezug auf die Ermittlung verbessert zu werden, indem unter Rückmeldung Ermittlungen für eine Anzahl von verschiedenen Kopfformen gemacht werden, um die Ermittlungskapazität des Algorithmus auf eine beliebige Kopfform anzupassen.

7. System nach Anspruch 5 oder 6, wobei der Klassifizierungsalgorithmus auf Merkmalen von künstlichen neuronalen Netzen basiert.

8. System nach einem der Ansprüche 5-7, wobei beabsichtigt ist, dass eine Information über die Richtung des Kopfes und Änderungen darin verwendet wird, um das Vorhandensein von Unachtsamkeit auf Seiten der Person zu ermitteln.

9. Kraftfahrzeug (1), das ein System (I) nach einem der Ansprüche 5-8 beinhaltet.

10. Computerprogramm (P) zum Ermitteln der Richtung des Kopfes einer Person, wobei das Computerprogramm (P) Programmcode beinhaltet, der, wenn er von einer elektronischen Steuereinheit (100) oder einem anderen mit der elektronischen Steuereinheit (100) verbundenen Computer (500) ausgeführt wird, der elektronischen Steuereinheit (100) ermöglicht, die Schritte nach den Ansprüchen 1-4 durchzuführen.

11. Computerprogrammprodukt, das ein digitales Speichermedium umfasst, das das Computerprogramm (P) nach Anspruch 10 speichert.

## Revendications

1. Procédé pour déterminer des informations concernant la direction de la tête, dans lequel ladite tête appartient à un conducteur de véhicule présent dans un compartiment pour conducteur de véhicule, comprenant les étapes consistant à :
créer (S1) une image de ladite tête comprenant une vue souhaitée de la tête ;
créer (S2) une image de données de profondeur (D1, D2, D3) de ladite tête ;
extraire (S3) des données de profondeur à partir de points définis (DP) sur l'image de données de profondeur de la tête ; **caractérisé par** les étapes consistant à
déterminer la position de ladite tête dans l'image de données de profondeur créée (D1, D2, D3), dans lequel l'étape de détermination (S4) de la position de ladite tête dans l'image de données de profondeur créée (D1, D2, D3) comprend l'étape consistant à créer un contour de tête bidimensionnel en tant que base pour ladite détermination, dans lequel ladite image de contour est utilisée pour correspondre à un modèle de tête binaire,
déterminer (S4) ladite direction de la tête en utilisant un algorithme de classification utilisant lesdites données de profondeur extraites à partir desdits points définis (DP) sur l'image de données de profondeur de la tête, dans lequel ladite classification de la direction de la tête comprend essentiellement une tête droite, tournée vers la droite, tournée vers la gauche, tournée vers le haut et/ou tournée vers le bas et dans lequel lesdits points définis (DP) consistent en un ensemble de points répartis selon un motif prédéterminé et dans lequel les coordonnées desdits points définis (DP) sont calculées sur la base des positions connues pour le modèle de tête déterminé.

2. Procédé selon la revendication 1, dans lequel ledit algorithme de classification est, pendant une phase d'apprentissage, continuellement amélioré en termes de ladite détermination en effectuant, sous rétroaction, des déterminations pour un nombre de formes de tête différentes afin d'adapter la capacité de détermination de l'algorithme à toute forme de tête.

3. Procédé selon la revendication 1 ou 2, dans lequel ledit algorithme de classification est basé sur des caractéristiques de réseaux de neurones artificiels.

4. Procédé selon l'une quelconque des revendications 1 à 3, dans lequel des informations concernant ladite direction de la tête et des changements de celle-ci sont utilisées pour déterminer la présence d'une inattention de la part de ladite personne.

5. Système (I) pour déterminer des informations concernant la direction de la tête, dans lequel ladite tête appartient à un conducteur de véhicule présent dans un compartiment pour conducteur de véhicule, comprenant des moyens (110) pour créer une image de ladite tête comprenant une vue souhaitée de la tête, des moyens (100, 110) pour créer une image de données de profondeur (D1, D2, D3) de ladite tête ; des moyens (100, 106) pour extraire des données de profondeur à partir de points définis (DP) sur l'image de données de profondeur (D1, D2, D3) de la tête ; des moyens (100, 108) pour utiliser lesdites données de profondeur extraites afin de déterminer ladite direction de la tête en utilisant un algorithme de classification, et dans lequel ladite classification de la direction de la tête comprend essentiellement une tête droite, tournée vers la droite, tournée vers la gauche, tournée vers le haut et/ou tournée vers le bas, **caractérisé en ce que** le système comprend en outre des moyens (100, 104) pour déterminer la position de ladite tête dans l'image de données de profondeur créée (D1, D2, D3), dans lequel lesdits moyens (100, 104) pour déterminer la position de ladite tête dans l'image de données de profondeur créée (D1, D2, D3) comprennent des moyens pour créer un contour de tête bidimensionnel comme base pour ladite détermination, dans lequel ladite image de contour est utilisée pour correspondre à un modèle binaire de l'image et dans lequel lesdits points définis (DP) consistent en un ensemble de points répartis selon un motif prédéterminé et dans lequel les coordonnées desdits points définis (DP) sont calculées sur la base des positions connues pour le modèle de tête déterminé.

6. Système selon la revendication 5, dans lequel ledit algorithme de classification est conçu, pendant une phase d'apprentissage, pour être continuellement amélioré en termes de ladite détermination en effectuant, sous rétroaction, des déterminations pour un nombre de formes de tête différentes afin d'adapter la capacité de détermination de l'algorithme à toute forme de tête.

7. Système selon la revendication 5 ou 6, dans lequel ledit algorithme de classification est basé sur des caractéristiques de réseaux de neurones artificiels.

8. Système selon l'une quelconque des revendications 5 à 7, dans lequel des informations concernant ladite direction de la tête et des changements de celle-ci sont destinées à être utilisées pour déterminer la présence d'une inattention de la part de ladite personne.

9. Véhicule motorisé (1) contenant un système (I) selon l'une quelconque des revendications 5 à 8.

10. Programme informatique (P) pour déterminer la direction de la tête d'une personne, dans lequel ledit programme informatique (P) contient un code de programme qui, lorsqu'il est exécuté par une unité de commande électronique (100) ou un autre ordinateur (500) connecté à l'unité de commande électronique (100), permet à l'unité de commande électronique (100) d'effectuer les étapes selon les revendications 1 à 4.

11. Produit de programme informatique comprenant un support de stockage numérique qui stocke le programme informatique (P) selon la revendication 10.
